# EUROPEAN PATENT SPECIFICATION

(11) **EP 1 105 155 B1**
(45) Date of publication and mention of the grant of the patent: **24.11.2004**
(21) Application number: 99940299.3
(22) Date of filing: 19.08.1999
(51) Int. Cl.: A61K 39/00, A61K 38/00, C07K 14/47, C07K 1/00

(54) **VACCINE COMPRISING CYTOKINE-INDUCED HEAT SHOCK PROTEINS (HSP)**
IMPFSTOFF ENTHALTEND ZYTOKIN-INDUZIERTE HEAT SHOCK PROTEINE (HSP)
VACCIN CONTENANT DES PROTEINES DE CHOC THERMIQUE (HSP) INDUITES PAR DES CYTOKINES

(30) Priority: 19.08.1998 GB 9818133
(43) Date of publication of application: 13.06.2001
(73) Proprietor: Immunobiology Limited, Cambridge CB2 4AT (GB)
(72) Inventor: COLACO, Camilo, Anthony, Leo, Selwyn, Cambridge CB2 2JN (GB); COLACO, Susanna, Mary, Cambridge CB2 2JN (GB)
(74) Representative: Pattullo, Norman
(86) International application number: PCT/GB1999/002607
(87) International publication number: WO 2000/010597

(56) References cited:
- WO-A-95/24923
- WO-A-97/10001
- US-A- 5 750 119
- BLACHERE N E ET AL: "Heat shock protein vaccines against cancer" JOURNAL OF IMMUNOTHERAPY,US,RAVEN PRESS, NEW YORK, vol. 14, page 352-356 XP002037576 ISSN: 1053-8550
- METZ K ET AL: "Interleukin-4 upregulates the heat shock protein Hsp90alpha and enhances transcription of a reporter gene coupled to a single heat shock element." FEBS LETTERS, (1996 APR 29) 385 (1-2) 25-8. , XP002127261
- WACHLIN, G. ET AL: "Cytokine - induced stress response in pancreatic islets from diabetes-prone BB and MHC -congenic BB.1A rats." EXPERIMENTAL AND CLINICAL ENDOCRINOLOGY & DIABETES, (1997) VOL. 105, NO. 4, PP. A46. MEETING INFO.: SATELLITE MEETING TO THE 16TH INTERNATIONAL DIABETES FEDERATION CONGRESS, JOINT MEETING WITH THE EUROPEAN ASSOCIATION FOR THE STUDY OF DIABETES ISLET , XP002127262

## Description

The present invention relates to a vaccine and a method for producing a vaccine.

### BACKGROUND OF THE INVENTION

An important component of any human immune response is the presentation of antigens to T cells by antigen presenting cells (APCs) such as macrophages, B cells or dendritic cells. Fragments of foreign antigens are presented on the surface of the macrophage in combination with Major histocompatibility complex (MHC) molecules, in association with helper molecules, such as CD4 and CD8 molecules. Such "antigenic fragments", presented in this way, are recognised by the T cell receptor of T cells, and the interaction of the antigen with the T cell receptor results in antigen-specific T cell proliferation, and secretion of lymphokines by the T-cells.

The nature of the antigenic fragment presented by the APCs is critical in establishing immunity. Heat shock proteins (HSPs) form a family of highly conserved proteins that are widely distributed throughout the plant and animal kingdoms. On the basis of their molecular weights, HSPs are grouped into six different families: small (hsp 20-30kDa); hsp40; hsp60; hsp70; hsp90; and hsp100. Although HSPs were originally identified in cells subjected to heat stress, they have been found to be associated with many other forms of stress such as infections, and are thus more commonly known as "stress proteins" (SPs).

Members of the mammalian hsp90 family include cytosolic hsp90 (hsp83) and the endoplasmic reticulum counterparts hsp90 (hsp83), hsp87, Grp94 (Erp99) and gp97. See for instance, Gething et al. (1992) *Nature* 355:33-45. Members of the hsp70 family include cytosolic hsp70 (p73) and hsp70 (p72), the endoplasmic reticulum counterpart BiP (Grp78), and the mitochondrial counterpart hsp70 (Grp75). Members of the mammalian hsp60 family have only been identified in the mitochondria.

SPs are ubiquitous within cells, one of the roles of SPs is to chaperone peptides from one cellular compartment to another and to present peptides to the MHC molecules for cell surface presentation to the immune system. In the case of diseased cells, SPs also chaperone viral or tumour-associated peptides to the cell-surface. Li and Sirivastave (1994) *Behring Inst. Mitt,* 94: 37-47 and Suzue et al. (1997) *Proc*.*Natl*.*Acad*.*Sci*. *USA* 94: 13146-51. The chaperone function is accomplished through the formation of complexes between SPs and proteins and between SPs and viral or tumour-associated peptides in an ATP-dependent reaction. SPs bind a wide spectrum of peptides in an ATP dependent manner. The bound peptides appear to be a random mix of peptides. The mixtures and exact natures of the peptides have not been determined. The association of SPs with various peptides has been observed in normal tissues as well and is not a tumour-specific phenomenon. See Srivastava (1994) *Experimentia* 50: 1054-60.

For instance, expression of hsp26, hsp60, hsp70 and hsp90 on the surface of human chronic myeloid leukemia (CML) cells from patients has been observed, Chant et al. (1995) *Br*.*J*.*Haematol*. 90: 163-8. Cell surface expression of hsp70 has been detected on normal, pre-malignant and malignant human oral mucosa, Kaur et al. (1998) *Oral Oncol*. 34: 93-8. A correlation of hsp70 expression with clinico-pathological features showed a positive association with the severity of dysplasia in oral mucosal epithelium.

Ito et al. (1998) *J*.*Oral*.*Pathol*.*Med*. 27: 18-22, reported that they examined 24 specimens of squamous cell carcinoma of the tongue and found that, although SP immuno-histochemistry revealed changes in expression during tumorigenesis, there was no observed correlation with other clinical features studied (survival period, stage, lymph node metastasis, histological grade or p53 immuno-staining)

It is currently believed that the antigenicity of SPs results not from the SP per se, but from the complex of peptide associated with the SP. This conclusion is based on a number of characteristics of the complexes. There are no differences in the structure of SPs derived from normal and tumour cells. Certain complexes lose their immunogenicity upon treatment with ATP, Udono et al. (1993) *J*.*Exp*.*Med*. 178: 1391-96. Such loss of immunogenicity is due to dissociation of the complex into its SP and peptide components.

In a therapeutic context, it has been proposed to use SP-antigen complexes as vaccines. In particular, US Patent No. 5,750,119 to Srivastava discloses a multi-step, cancer patient-specific method for inhibiting the poliferation of a tumour in a mammal, by (a) removing tumour from the mammal; (b) isolating all complexes from the tumour cells and (c) administering the isolated complexes back to the mammal in order to stimulate a tumour-specific immune response. Hsp70-peptide, hsp90-peptide and gp96-peptide complexes are itemised as complexes having particular vaccine utility. Nevertheless, in the practice of the method disclosed by Srivastava, it is not considered necessary or even practical to isolate a specific peptide involved or even the particular complex in eliciting the immune response.

WO 97/10000 and WO 97/10001 disclose that a mixture of heat shock proteins (HSPs) isolated from cancer cells or virally infected cells are capable eliciting protective immunity or cytotoxic T lymphocytes to the cognate tumour or viral antigen. However, in contrast, HSPs isolated from normal cells are unable to elicit such immunity. It is now thought that HSPs are not immunogenic *per se,* but are able to elicit immunity because of their association with tumour or virus specific antigenic peptides that are generated during antigen processing. Specifically, the peptides associated with the HSPs are immunogenic, and are presented to the T cells. HSPs stripped of associated peptides lose their immunogenicity (Udono, H. and Srivastava, P. K., Journal of Experimental Medicine, *178*, page 1391 *ff*, 1993). To date, the nature of these peptides has not been determined.

The immunogenicity of HSP preparations depends upon the presence of phagocytic cells, such as macrophages and other APCs. It is now thought that HSPs are taken up by macrophages, and those peptides associated with the HSPs are then presented by MHC class I molecules of the macrophage. In this way, a T cell response is initiated.

The use of HSP proteins as vaccine components has been disclosed in WO 95/24923 and WO 98/34641. HSPs isolated from tumour cells or viral infected cells have been suggested as a source of antigenic peptides, which could then be presented to T cells. This necessitates the production and purification of HSPs from such cells. In order to help the purification, cells may be treated by heat shock or other stresses, to increase intracellular levels of the SPs. However, it is not presently know if such induced SPs will form immunogenic complexes with heterologous peptides as do constitutively expressed HSPs.

The stimulation of cells by heat shock produces a general increase in the level of heat shock proteins. Ideally, it would be desirable to stimulate the production of only a subset of HSPs, which are especially immunogenic. Such a subset of HSPs would be particularly suitable for use in the production of a vaccine. A number of other stresses such as osmotic stress, oxidative stress or heavy-metal treatment are known to produce closely related HSPs. However, at present, there is no way to specifically stimulate cells to produce such a subset of HSPs with enhanced immunogenicity.

### SUMMARY OF THE INVENTION

Therefore, in a first aspect, the present invention provides a method for producing a vaccine, comprising the steps of:
a) treating virally infected, recombinant or cancerous cells with a cytokine;
b) extraction of heat shock proteins from the treated cells; and
c) use of the extracted heat shock proteins in the preparation of a vaccine.

It is surprising that the treatment of virally infected, transformed or cancerous cells with cytokines, particularly interferon-α, produces HSPs which are more immunogenic than HSPs derived from cells which have been heat shocked. The most notable aspect of immunity elicited by cytokine-induced HSPs is the long-term memory compared to that induced by immunisation by other HSP subsets.

The term "vaccine" as used herein, refers to any composition which stimulates the immune system such that it can better respond to subsequent infections. It will be appreciated that a vaccine usually contains an immunogenic determinant and an adjuvant, which non-specifically enhances the response to that determinant.

Preferably, the immunogenic determinant for the present invention is delivered in combination with an adjuvant. Suitable adjuvants are readily apparent to the person skilled in the art, such as Freund's complete adjuvant, Freund's incomplete adjuvant, Quil A, Detox, ISCOMs or squalene. However, it will be appreciated that the vaccine of the present invention may also be effective without an adjuvant. Such a vaccine may be given by any suitable means, such as orally, or by injection.

The term "heat shock protein", as used herein, is standard in the art, and includes those proteins which are induced when a cell is subjected to heat stress, such as HSP70 and GRP96. These proteins play a role in the protection of other cellular proteins from the effects of heat stress. Heat shock proteins (HSPs) are also expressed under normal cellular conditions, where they play a role in protein folding. The family of HSPs includes stress proteins (SPs) and the members of this family of proteins that are both constitutively expressed as well as those induced by a variety of other stresses including enviromental insults and oxidative and osmotic stresses. The family of HSPs is defined primarily on amino acid sequence homologies.

Eucaryotic cells contain a number of families of HSPs, such as the HSP90 family, the HSP70 family, the HSP60 family and the GRP96 family (endoplasmin). These families are named on the basis of the size of the peptides which they encode. The families are highly conserved between species. In addition, many bacteria also express homologues of eucaryotic proteins. Preferably the vaccine contains at least one HSP derived from the cytoplasm of the virally infected, recombinant or cancerous cell, and one that is membrane bound. We particularly prefer that the HSP70, HSP90 and the GRP96 families of proteins are used as immunogenic determinants in the present invention, with HSP70 and GRP96 most preferred. Preferably the immunogenic proteins have greater than 25% homology and/or 20% identity at the amino acid level to the heat-induced HSP protein families.

The term "cytokine", as used herein, is standard in the art, and refers to an intercellular chemical signal. The present invention requires that the cytokine that is used is able to stimulate the presence of HSPs within virally infected or tumour cells. We prefer that the cytokine is an interferon, especially interferon-α (IFN-α). However, it will be appreciated that any suitable cytokine which is able to specifically induce the upregulation of a subset of immunogenic HSPs is included in the present invention. These cytokines include, but are not limited to, interferon-β (IFN-β), tumour necrosis factor α (TNFα), interleukins 1 and 6 (IL1 and IL6).

Treatment of virally infected, recombinant or cancer cells with IFN-α elicits HSPs that are more immunogenic, by weight, than those HSPs derived from cells that have been heat shocked, producing higher titres of antibodies and/or frequencies of cytotoxic T-cells. It will be appreciated that these HSPs carry the antigenic peptides derived from the cancer cell or carry the fragments of the viral peptides. Without being constrained by theory, it is thought that cytokine treatment works either to specifically induce those HSPs most able to interact with peptides, or to induce those HSPs which are most easily phagocytosed by APCs, or both. The cytokine may also act to increase levels of viral or tumour peptides, which are then able to interact with the HSPs and, thus, are suitable for effective stimulation of the immune system.

In the case of treatment with other cytokines, the present invention includes any suitable method of cytokine treatment which produces a subset of HSPs that are more immunogenic, by weight, than those produced by heat shock alone. Comparative immunogenicity can be determined by *in vivo* testing on animal models. Other suitable methods will be readily apparent to the person skilled in the art, 'Current Protocols in Immunology', Wiley Interscience, 1997.

The tumour-derived, transformed or virally infected cells are preferably treated with the cytokine *in vitro*. Cytokines are suitably used at 0.5-1000 international units (i.u)/ml of media, preferably about 1-500 i.u/ml. Specifically, for IFN-α we prefer that cells are treated with 10-500 i.u./ml and are then cultured for 10-16 hours. Alternatively, cells may be grown on cytokine-producing feeder-monolayers or induced to produce endogenous cytokines. However, it will be appreciated that some cells are more sensitive than other, and thus a greater or lesser amount of cytokine may be needed. For example, IFN-α is effective at 0.1-2 pg per ml of media with an ECV infected fibroblast cell line 2D9 but at 1-100 pg with the B6 cell line Daudi. Moreover, the incubation time of cells with cytokines is also variable. We prefer that a 6-18 hour exposure time is used, but this time may be reduced to 0.5-4 hours in some cases and still be effective.

The means to test for optimum cytokine levels and incubation period are readily available to the person skilled in the art. However, it will be appreciated that the exact level of cytokine is not crucial to the invention, as long as the treatment stimulates the production of the desired immunogenic HSPs within the treated cells. Similarly, the other conditions of treatment, such as the length of exposure to cytokine, cell incubation media and temperature of incubation are not essential features of the present invention and may be varied depending upon the exact nature of the cell population and cytokine that is used. Means to vary and optimise these parameters will be readily apparent to the person skilled in the art.

Preferably the cytokine is isolated from the same organism as the cell which is to be treated. Treatment of cells with cytokines from the same organism provides optimum stimulation of HSPs. However, the use of cytokines from other species or individuals may also be useful in up-regulation of the HSP levels in cells, to provide suitable HSPs for use in the present invention.

The terms 'virally infected', 'recombinant' and 'tumour' cells are standard in the art. Cells suitable for use in the present invention include any form of tumour cell, cells infected with any virus and cells transformed to express heterologous proteins or peptides. Such cells contain antigens which are not usually present in uninfected or normal cells or cell lines, or which are not normally presented to the immune system. Fragments of such peptides which interact with HSPs are able to stimulate the body's immune response to such abnormal cells. In the case of recombinant cell lines, the heterologous antigens expressed include any molecules to which an immune response is desired, including protozoan, parasitic, fungal and bacterial antigens.

Any suitable virally infected, recombinant or tumour cell can be used in the present invention, to provide a source of HSP. In the case of tumour cells, we prefer that the cells used for extraction of HSPs are cells derived directly from the tumour cells, although transformed cells are also appropriate. Methods for the transformation of cells extracted from tumours, for example, are well known and standard in the art. Furthermore, means for isolating tumorogenic or virally infected cells are well known, and may involve such techniques as biopsies, surgery, use of blood cells or cell scrapings from the throat or mouth. Recombinant cells can be produced by transfection, retroviral infection and other methods well known in the art. Other methods for the isolation of tumorogenic or virally infected cells will be readily apparent to the person skilled in the art.

In addition, we prefer that virally infected cells are obtained by infection of an appropriate cell line with the desired virus *in vitro*. Cells infected in this way can then be stimulated to produce antigenic HSPs, suitable for vaccination against that virus. This includes recombinant viruses that carry antigenic epitopes from a heterologous source such as those used to produce recombinant vaccines. These also include all types of transfected recombinant cells used to produce recombinant vaccines, such as mammalian cell lines transfected with recombinant vectors by standard methods in the art such as electroporation, liposome fusion and calcium phosphate. Furthermore, the invention also includes the use of HSPs from eucaryotic cells that respond to treatment of cytokines, including those expressing homologous, and recombinant cell lines expressing heterologous antigens. While the antigens are predominantly proteins and peptides, they can also include carbohydrate, nucleic acid and lipid moieties that bind HSPs.

It will be appreciated that HSPs derived from tumour cells, for example, will in general only be able to provide immune protection against the specific tumour from which the cells were derived. In order to provide the best vaccine protection, it would therefore be desirable to combine HSP preparations derived from a number of different tumours. This could provide immune protection against the full range of tumours, by exposing the immune system to antigens from each different type of tumour cell. Alternatively multiple tumour antigens, such as melanoma-associated antigens (MAGEs) and prostate specific antigen (PSA), can be expressed in recombinant cells which are then used to produce the cytokine induced HSPs. Similar arguments apply equally to antigens derived from virally infected cells. In the case that HSPs from different tumours are combined to form a vaccine, then we prefer that the HSP preparations are derived from the same species as the organism which is to be vaccinated. In the case of recombinant antigens and viral antigens heterlogous HSP vaccines are equally preferred.

Moreover, it will be appreciated that a patient-specific cancer vaccine is also encompassed by the invention. The vaccine can be prepared by isolating a specific patient's tumour cells, for example, and treating the cells with a cytokine to stimulate the production of HSPs. The HSPs produced in this way could then be purified and be used in a vaccine, to stimulate the patient's specific immune response to his or her own tumour.

It will further be appreciated that different tumours may share the same antigenic peptides, in which case a HSP preparation derived from one specific tumour line would provide immune protection against other tumours, which also share the same antigenic peptide fragment. Recombinant cells expressing tumour antigens can also be used to produce effective HSP vaccine preparations.

The extraction and purification of HSP proteins from virally infected cells or tumour cells is standard in the art. Suitable methods include disruption of treated cells by homogenisation or sonication, followed by centrifugation to obtain a crude HSP preparation in the supernatant. In addition, ConA fractionation or ADP binding columns can also be used to purify the HSPs. Other suitable methods for the extraction of HSPs are readily available to the person skilled in the art, see for example those described in WO 97/10000 and WO 97/10001.

It will be appreciated that the present invention also includes methods of producing and isolating specific immunogenic HSPs from cancer cells, recombinant cells or virally infected cells, essentially as described above. HSPs derived in this way may be used to isolate samples of antigenic peptides, for example.

### Example 1

### Preparation of cytokine-induced SPs.

One specific example of the HSP preparation process is as follows. Cancerous or virally infected cells are incubated in a serum free media, such as RPMI (Sigma), with a suitable cytokine overnight. Cells are then washed in serum-free media, followed by a wash in phosphate buffered saline (PBS). The cells are then re-suspended in homogenisation buffer. The homogenisation buffer may be a hypotonic buffer, such as 10 mM phosphate pH 7.4 with 2mM MgCl₂, after which the cells are then disrupted using a cell homogeniser (e.g. a Dounce or Potter homogeniser, Ultraturrax or Waring blender). Alternatively, the homogenisation buffer may contain detergent, such as PBS with 0.5% Tween, the detergent concentration being between 0.1-1% and suitable to solubilise the cell membrane. The cell lysate is then treated by centrifugation, typically 3-5000 x g for 5 minutes, to remove the nuclei and cell debris, followed by a high speed centrifugation step, typically 100,000g for 15-30 minutes.

The supernatant thus obtained is processed to give a protein complex suitable for use in a vaccine. This can be done simply by ammonium sulphate precipitation which uses a 50-70% ammonium sulphate cut. Specifically, 50 % (w/w) ammonium sulphate is added at 4°C, the precipitate is discarded, followed by the addition of more ammonium sulphate to bring the concentration to 70 %. The protein precipitate is harvested by centrifugation, and then dialysed into an appropriate physiological, injectable buffer, such as saline, to remove the ammonium sulphate before use. It will be appreciated that the HSPs isolated in this way are not purified to homogeneity, but are nevertheless suitable for use as a vaccine component.

If more purified HSP preparation is required, then the HSPs may be purified from the supernatant by affinity chromatography on matrices carrying adenosine diphosphate, such as ADP-agarose or ADP-sepharose. These methods are standard in the art, and are outlined in WO 97/10000, WO 97/10001 and WO 97/10002.

HSPs may be used at any suitable concentration. We prefer that the amount of HSP70 and HSP90 complex that is administered is in the range of 10-600 µg, preferably 10-100 µg, most preferably 25 µg. For HSP90 complexes we prefer that levels used are 50-5000 µg, preferably 100 µg.

In order to determine the immunogenicity of stress protein-peptide complexes, T cell proliferation assays may be used. Suitable assays include the mixed-lymphocyte reaction (MLR), assayed by tritiated thymidine uptake, and cytotoxicity assays to determine the release of ⁵¹Cr from target cells. Both of these assays are standard in the art (see 'Current Protocols in Immunology', Wiley Interscience, 1997). Alternatively, antibody production may be examined, using standard immunoassays or plaque-lysis assays, or assessed by_ interuterine protection of a foetus. (see 'Current Protocols in Immunology', *supra*).

### Example 2

### Immunisation with cytokine induced HSPs; immunity in vaccine recipient

Mouse 3T3 cells infected with VSV or recombinant 3T3 cells expressing with VSV G-protein were treated overnight with 5pg/ml interferon-α. HSPs were prepared from detergent lysates (0.25% Triton) by fractionation of the cellular extracts to produce a 50-70% ammonium sulphate fraction. Mice and rabbits were vaccinated with 1-10 micrograms of the stress-protein containing extract in phosphate buffered saline and boosted with identical vaccine dosages a month after the primary injection. Induction of immunity to VSV was assayed using an ELISA to detect antibody against VSV G-protein. Antibody titres of 1:1-10,000 were routinely obtained. Cytotoxic T-cell activity directed against VSV G-protein could also be detected in the HSP-immunised mice. Challenge of the rabbits with fixed VSV particles at 6, 12 and 18 months periods after the initial immunisations resulted in the production of good antibody responses with titres of 1:1-10 000 indicating good memory responses in the immunised animals.

### Example 3

### Immunisation with cytokine induced HSPs; protection against live challenge.

Mouse 3T3 cells expressing Sheep pestivirus E1/2 and NS3 proteins were treated overnight with interferon-α and HSPs were prepared from detergent lysates as described in Example 1. Sheep were vaccinated with 10-50 micrograms of HSPs in phosphate buffered saline and boosted with identical vaccine dosages a month after the primary injection. Production of antibodies against E1/2 proteins was detected by Western blotting. Challenge of animals with live virus (3 x 10⁶) showed maximum antibody responses at 1-2 weeks compared to 4-5 weeks in the controls and initial studies showed prevention of transmission of virus to the foetus in pregnant ewes. Most interestingly, the high antibody titres of >1 in 100 000 appeared to completely inhibit viral replication in immunised animals as assayed by the detection of processed E1/2 antigens or core viral antigens in the serum of immunised animals.

## Claims

1. An in-vitro method for producing a vaccine, **characterised in that** it comprises the steps of:
a) treating virally infected cells, recombinant cells expressing a heterologous antigen to which a response is desired, or cancerous cells with a cytokine in an amount sufficient to induce the production of heat shock related stress proteins;
b) extracting the cytokine-induced heat shock proteins from the treated cells;
and
c) using the extracted heat shock proteins in the preparation of a vaccine.

2. A method as claimed in claim 1 **characterised in that** the vaccine produced contains an adjuvant.

3. A method as claimed in either of claims 1 or 2, **characterised in that** the cytokine is selected from an interferon, a tumour necrosis factor and an interleukin.

4. A method as claimed in claim 3, wherein the cytokine is interferon α or β.

5. A method as claimed in claim 3, wherein cytokine is interleukin 1 or 6.

6. A method as claimed in claim 3, wherein the cytokine is tumour necrosis factor α.

7. A method as claimed in any one of claims 1 to 6, **characterised in that** the cells to be treated with the cytokine are derived from the same species as that of the patient to which the vaccine produced is to be administered.

8. A method as claimed in any one of the preceding claims, **characterised in that** the cells to be treated with the cytokine are obtained from the patient to which the vaccine is to be administered.

9. A method as claimed in any one of the preceding claims, **characterised in that** the cytokine is isolated from the same organism as the cell which is to be treated.

10. A method as claimed in any one of the preceding claims, **characterised in that** the virally infected cells are obtained by infection of an appropriate cell line with the desired virus in-vitro.

11. A vaccine composition for administration to a patient, **characterised in that** it contains a vaccine obtained according to the method of any one of claims 1 to 10.

12. Use of a cytokine induced heat shock protein from a virally infected cell, a recombinant cell expressing a heterologous antigen or a cancerous cell in the manufacture of a vaccine to elicit an immune response.

## Patentansprüche

1. Eine in vitro-Methode zur Herstellung eines Impfstoffs, **dadurch gekennzeichnet, dass** sie die folgenden Schritte enthält:
a) Behandeln von mit einem Virus infizierten Zellen, rekombinanten Zellen, die ein heterologes Antigen exprimieren, auf das eine Reaktion erwünscht ist, oder von Krebszellen mit einem Zytokin in einer Menge, die ausreicht, um die Herstellung von mit Hitzeschock verbundenen Stressproteinen zu induzieren,
b) Extrahieren von zytokininduzierten Hitzeschockproteinen aus den behandelten Zellen und
c) Verwenden der extrahierten Hitzeschockproteine bei der Vorbereitung eines Impfstoffs.

2. Methode gemäß Anspruch 1, **dadurch gekennzeichnet, dass** der hergestellte Impfstoff ein Hilfsmittel enthält.

3. Methode gemäß Anspruch 1 oder 2, **dadurch gekennzeichnet, dass** das Zytokin aus einem Interferon, einem Tumor-Nekrose-Faktor und einem Interleukin ausgewählt ist.

4. Methode gemäß Anspruch 3, wobei das Zytokin Interferon α oder β ist.

5. Methode gemäß Anspruch 3, wobei das Zytokin Interleukin 1 oder 6 ist.

6. Methode gemäß Anspruch 3, wobei das Zytokin der Tumor-Nekrose-Faktor α ist.

7. Methode gemäß einem der Ansprüche 1 bis 6, **dadurch gekennzeichnet, dass** die mit Zytokin zu behandelnden Zellen von derselben Spezies abstammen, wie die des Patienten, dem der hergestellte Impfstoff verabreicht werden soll.

8. Methode gemäß einem der vorhergehenden Ansprüche, **dadurch gekennzeichnet, dass** die mit Zytokin zu behandelnden Zellen von dem Patienten erhalten werden, dem der Impfstoff verabreicht werden soll.

9. Methode gemäß einem der vorhergehenden Ansprüche, **dadurch gekennzeichnet, dass** das Zytokin aus demselben Organismus isoliert wird, wie die zu behandelnde Zelle.

10. Methode gemäß einem der vorhergehenden Ansprüche, **dadurch gekennzeichnet, dass** die mit einem Virus infizierten Zellen durch eine Infektion einer geeigneten Zelllinie mit dem gewünschten Virus in vitro erhalten werden.

11. Eine Impfstoffzusammensetzung zur Verabreichung an einen Patienten, **dadurch gekennzeichnet, dass** sie einen Impfstoff enthält, der gemäß der Methode aus einem der Ansprüche 1 bis 10 erhalten wurde.

12. Die Verwendung eines zytokininduzierten Hitzeschockproteins aus einer mit einem Virus infizierten Zelle, einer ein heterologes Antigen exprimierenden rekombinanten Zelle oder einer Krebszelle bei der Fertigung eines Impfstoffs zum Auslösen einer Immunreaktion.

## Revendications

1. Un procédé in vitro destiné à la production d'un vaccin, **caractérisé en ce qu'**il comprend les étapes de :
a) traiter des cellules infectées de façon virale, des cellules recombinées exprimant un antigène hétérologue contre lequel une réponse est souhaitée, ou des cellules cancéreuses avec une cytokine en quantité suffisante pour induire la production de protéines de stress lié au choc thermique ;
b) extraire les protéines de choc thermique induites par la cytokine provenant des cellules traitées ;
et
c) utiliser les protéines de choc thermique extraites dans la préparation d'un vaccin.

2. Un procédé tel que revendiqué dans la revendication 1 **caractérisé en ce que** le vaccin produit contient un adjuvant.

3. Un procédé tel que revendiqué dans l'une ou l'autre des revendications 1 et 2, **caractérisé en ce que** la cytokine est sélectionnée parmi un interféron, un facteur de nécrose tumorale et une interleukine.

4. Un procédé tel que revendiqué dans la revendication 3, dans lequel la cytokine est l'interféron α ou β.

5. Un procédé tel que revendiqué dans la revendication 3, dans lequel la cytokine est l'interleukine 1 ou 6.

6. Un procédé tel que revendiqué dans la revendication 3, dans lequel la cytokine est le facteur de nécrose tumorale α.

7. Un procédé tel que revendiqué dans n'importe laquelle des revendications 1 à 6, **caractérisé en ce que** les cellules devant être traitées avec la cytokine sont dérivées de la même espèce que celle du patient auquel le vaccin produit doit être administré.

8. Un procédé tel que revendiqué dans n'importe laquelle des revendications précédentes, **caractérisé en ce que** les cellules devant être traitées avec la cytokine sont prélevées sur le patient auquel le vaccin doit être administré.

9. Un procédé tel que revendiqué dans n'importe laquelle des revendications précédentes, **caractérisé en ce que** la cytokine est isolée du même organisme que la cellule devant être traitée.

10. Un procédé tel que revendiqué dans n'importe laquelle des revendications précédentes, **caractérisé en ce que** les cellules infectées de façon virale sont obtenues par infection in vitro d'une lignée cellulaire appropriée avec le virus souhaité.

11. Une composition de vaccin destinée à être administrée à un patient, **caractérisée en ce qu'**elle contient un vaccin obtenu selon le procédé de n'importe laquelle des revendications 1 à 10.

12. Utilisation d'une protéine de choc thermique induite par la cytokine provenant d'une cellule infectée de façon virale, d'une cellule recombinée exprimant un antigène hétérologue ou d'une cellule cancéreuse dans la fabrication d'un vaccin pour provoquer une réponse immune.
